# EUROPEAN PATENT SPECIFICATION

(11) **EP 3 183 571 B1**
(45) Date of publication and mention of the grant of the patent: **24.02.2021**
(21) Application number: 14755632.8
(22) Date of filing: 18.08.2014
(51) Int. Cl.: G01N 33/04, G01N 33/14, G01N 21/35

(54) **DETERMINATION OF A CONSTITUENT RELATED PROPERTY OF A MULTI-CONSTITUENT SAMPLE**
BESTIMMUNG EINER BESTANDTEIL-BEZOGENEN EIGENSCHAFT EINER PROBE AUS MEHREREN BESTANDTEILEN
DÉTERMINATION D'UNE PROPRIÉTÉ LIÉE À UN CONSTITUANT D'UN ÉCHANTILLON MULTI-CONSTITUANT

(43) Date of publication of application: 28.06.2017
(73) Proprietor: FOSS Analytical A/S, 3400 Hillerød (DK)
(72) Inventor: NOERGAARD, Lars, 2900 Hellerup (DK); BAUM, Andreas, 2700 Broenshoej (DK); JUHL, Henrik Vilstrup, 4000 Roskilde (DK); HANSEN, Per Waaben, 2800 Kgs. Lyngby (DK); MIKKELSEN, Joern Dalgaard, 2800 Kgs. Lyngby (DK)
(86) International application number: PCT/EP2014/067549
(87) International publication number: WO 2016/026506

(56) References cited:
- BAUM ANDREAS ET AL: "Enzyme activity measurement via spectral evolution profiling and PARAFAC", ANALYTICA CHIMICA ACTA, vol. 778, 21 March 2013 (2013-03-21), pages 1-8, XP028592916, ISSN: 0003-2670, DOI: 10.1016/J.ACA.2013.03.029
- BAUM A: "Descriptive and predictive assessment of enzyme activity and enzyme related processes in biorefinery using IR spectroscopy and chemometrics", PH D THESIS TECHNICAL UNIVERSSITY DENMARK, , 1 May 2013 (2013-05-01), pages 1-124, XP007922909, Retrieved from the Internet: URL:http://orbit.dtu.dk/files/74249022/PhD _Thesis.PDF

## Description

The present invention relates to a method for the determination of a constituent related property of a multi-constituent sample and in particular to such a determination using chemometric analysis of electromagnetic radiation after it's interaction with the sample.

In the food industry for example, there is an increasing interest both from the consumer and the producer in obtaining more detailed information regarding one or more constituent related properties of a product, for example, going from total fat content to fatty acids profiling in meat and dairy products; going from total protein content to protein fractions(such as from milk protein to casein and whey fractions); going from total carbohydrate to sugar profiling in milk, wine and juice products; or identifying low concentration components, such as malic acid in wine and juice products or acetone in milk; or determining physical or functional properties of a food.

In the present context the phrase 'constituent related property' means a compositional, physical or functional property of a multi-constituent sample which is affected by one or more of the constituents of the sample. Similar phrases will have similar meanings.

Thus it is expected that information regarding a constituent related property may be obtained from measurements made on the constituent itself.

It is well known that different constituents of a multi-constituent product interact differently with optical radiation, particularly within the infra-red region of the electromagnetic spectrum, to produce more or less distinctive 'spectral fingerprints'. Stretching and bending vibrations of chemical bonds of sample constituents, for example, can provide characteristic absorption bands within the electromagnetic spectrum, particularly the near and mid infra red portions of the spectrum. Different particle size, which for ground cereal grains is related to hardness, may cause a sample to exhibit differential light scattering and/or absorption characteristics which again can be monitored through the interaction of optical radiation with the sample to provide information on a property of interest within the sample. Chemometric analysis of optical spectra which derive from the detection of electromagnetic radiation in one or more wavelength regions from within ultra violet to infra red portions of the electromagnetic spectra (referred to herein as 'optical radiation') after it has interacted with a sample is now commonly employed as a means to derive quantitative or qualitative information about a property of the sample. Chemometric analysis is a so-called 'indirect' technique, meaning that the constituent related information is not directly available from the recorded spectral data. Rather a calibration must be established by linking spectral features of reference samples with information regarding a property of interest of those samples, which information is obtained for each reference sample using other, typically direct, analysis techniques. However, advantageously, chemometric analysis offers the ability to mathematically extract the relevant information about the property of interest of the sample through the development of a model that subsequently can be used for quantitative property prediction of new samples as well as for detection of deviating samples not taken into account in the calibration samples.

BAUM ANDREAS ET AL: "Enzyme activity measurement via spectral evolution profiling and PARAFAC",ANALYTICA CHIMICA ACTA, vol. 778, 21 March 2013 (2013-03-21), pages 1-8, ISSN: 0003-2670, DOI:10.1016/J.ACA.2013.03.029, relates to monitoring changes of enzyme activity in real time by evolution profiling of Fourier Transform Infrared spectral fingerprints using parallel factor analysis.

Unfortunately, constituents with similar chemical bonds often produce similar or strongly overlapping spectral fingerprints. This will make it difficult to employ chemometric analysis of conventional optical spectra in order to determine properties of the sample which are related to these constituents.

According to the independent claim 1 there is provided a method of determining a concentration of a constituent of a multi-constituent sample comprising: subjecting the sample to a perturbation selected to induce a time dependent change in a wavelength dependent intensity of an electromagnetic wave after its interaction with the sample, the electromagnetic wave having wavelength components, the intensities of which change dependent on the perturbation induced time dependent change; recording a time-series of the wavelength dependent intensity of the electromagnetic wave following subjecting the sample to the perturbation; and determining the concentration of the constituent from the application of a calibration to the recorded time-series said calibration correlating the concentration with the time-series of the wavelength dependent intensity of the electromagnetic wave and being empirically derived from multivariate chemometric modelling of time-series wavelength dependent intensities of the electromagnetic wave recorded for each of a plurality of reference samples following subjecting each reference sample to the perturbation, each reference sample having a different known value of the concentration of the constituent.

By changing the known, static measurement situation into a dynamic measurement situation through inducing sample perturbations affecting only a constituent related to the sample property to be determined, either hardware or in-sample perturbations, then temporal evolution data may be employed to provide a characteristic temporal development profile and changes in its content associated only with the constituent of interest. Since it is a specific change in the measurement data which is now being employed even constituents which are present in low (even previously undetectable) concentrations may generate relatively large changes which allow their detection and a property determination, such as constituent concentration or constituent dependent physical or functional properties.

Moreover, by employing the dynamic time-resolved measurement data the method of the present invention can be made much more robust towards interferences and the amount of required calibration reference samples may be reduced by using advanced chemometric dynamic time-series modelling.

Physical or chemical perturbations are induced directly in the sample during measurement by means of, for example, temperature change, addition of a chemical, addition of one or more enzymes, salt or pH changes. In every event the in-sample perturbation employed will be selected to induce a physical or chemical change in the sample which manifests as a change in measurement data associated with the constituent related information of interest. A hardware perturbation is one which is applied external of the sample to cause perturbations in the sample, for example, to induce a movement of dispersed particles, such as molecules, in a fluid sample held in a sample presentation unit, comprising for example a cuvette, as the result of applying a current or magnetic field to the fluid sample.

In the following the invention is further explained and exemplified with reference to the figures, of which:
- Fig.1: Illustrates a typical mid-IR spectrum of milk;
- Fig. 2: Illustrates schematically a representative sample property monitor not according to the present invention;
- Figs. 3: Show exemplary temporal evolution profiles obtained using an analyser as shown in Fig. 2;
- Fig. 4: Show a PARAFAC kinetic mode retrieved from profiles as illustrated in Figs. 3;
- Fig. 5: Shows a calibration for K-casein in milk obtained by PARAFAC modelling of temporal evolution profiles exemplified in Fig.3; and

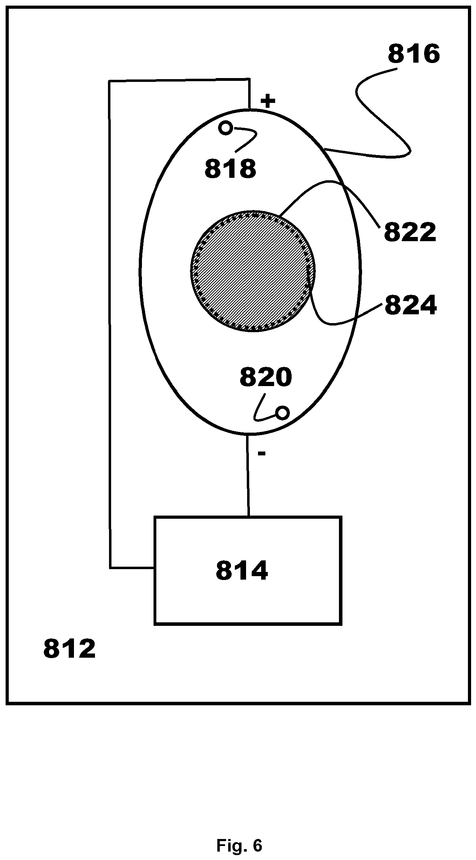

- Fig. 6: Shows schematically an example of a sampling unit not in accordance with the present invention which may be employed in a monitorof Fig. 1 for use in electrophoretic based determinations.

An embodiment of the present method will now be described by way of example only. According to this exemplary embodiment a determination of the amount of Kappa-casein ('K-casein') in milk is made as the constituent related sample property. This determination is made by analysing perturbation induced time dependent changes in a property of an electromagnetic wave, here intensities of wavelength components of the electromagnetic wave, after the wave has interacted with the milk.

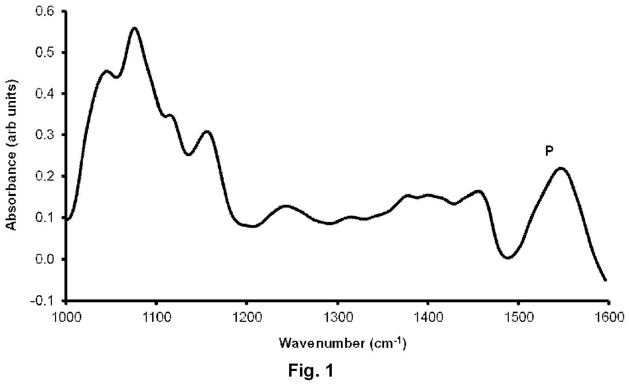

Considering now Fig. 1, a representative mid-infrared absorbance spectrum of milk in the wavenumber region 1000 cm⁻¹ to 1600 cm⁻¹ (corresponding to the wavelength region 10,000 nm to 6250 nm) is illustrated. The protein (P) related feature of the milk sample spectral fingerprint is identified. Casein is known to represent around 80% of the total protein content. Since, according to Beer's law, absorption of the electromagnetic energy (here mid-infrared energy) is proportional to the amount of absorbing component then detection of casein using chemometric analysis of conventional, static, optical spectra should be possible. However, as can be seen from Fig. 1, the protein spectral feature (P) shows no clearly distinguishable structure. Spectral features related to K-casein are essentially indistinguishable from those of the other proteins in milk. As will be appreciated by those skilled in the art a conventional chemometric model developed to predict K-casein content from such measurement data would, in these circumstances, be inaccurate.

It is well known that gelation of casein micelles with addition of the enzyme chymosin is the first step in the manufacture of cheese. The enzyme specifically removes the C-terminus of the K-casein causing destabilization and subsequent agglomeration of the casein micelles which agglomeration causes changes in the optical spectral fingerprint of the milk sample. By way of example only, using this enzyme as a chemical perturbation on a milk sample a time-series of spectral data can be recorded for each sample as a monitor of the temporal evolution of K-casein agglomeration. Two enzymatic preparations (chymosin preparations) were used in the present exemplary embodiment, namely CHYMAXPLUS™ and CHYMAXM1000™, both obtained from Chr. Hansen, Bøge Allé 10-12, DK-2970 Hørsholm, Denmark. CHYMAXM1000 shows highest specificity for K-casein and was therefore used to establish an empirical calibration as described below which links the sample property (here K-casein concentration) with time-series spectral data (here representing temporal evolution of spectral absorption). Both enzymatic preparations show very comparable spectral evolutions and act in a similar manner concerning the agglomeration of K-casein in milk.

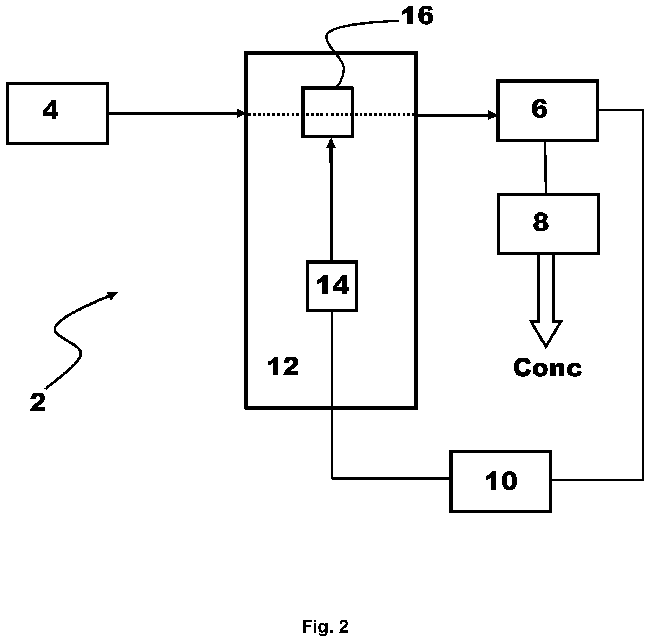

All electromagnetic spectral measurement data i may be recorded using a sample property monitor 2 as illustrated schematically in Fig. 2 and operating to record spectral data in the wavenumber region between 1000 cm⁻¹ to 1600 cm⁻¹. According to Fig. 2 such a sample property monitor 2 may comprise an output unit 4; a detection unit 6; a determinations unit 8; a control unit 10; and a sampling unit 12, which sampling unit 12 comprises a perturbations unit 14 and a sample presentation unit 16.

The output unit 4 i may be adapted to output an electromagnetic wave towards a multi-constituent liquid sample (here milk) in the sample presentation unit 16. The electromagnetic wave i may be a mid-infrared electromagnetic wave having wavelength components at least extending in the region between 6250nm (1600cm⁻¹) and 10000nm (1000cm⁻¹). It will be appreciated that generally the wavelengths of electromagnetic energy emitted by the output unit 4 are selected dependent on the constituent(s) within the sample which gives rise to the constituent related sample property being monitored.

The detection unit 6 may detect a property of the electromagnetic wave output from the output unit 4 after the wave interacts with the liquid sample in the sample presentation unit 16. The detection unit 6 may be configured to monitor an energy (wavelength or wavenumber) dependent intensity variation of the output electromagnetic wave that is induced by its interaction with the liquid sample and to provide this electromagnetic absorption spectral data as output measurement data. Here, and by way of example only, the detection unit 6 may be configured to detect the electromagnetic energy transmitted through the sample and may, as in the present example, comprise a Fourier Transform infrared (FTIR) spectrometer although other known spectrophotometric devices, such as a monochromator or detector diode array (DDA), which are adapted to provide an output of intensity indexed against energy (wavelength or wavenumber) may be substituted.

The determinations unit 8 may comprise a data processor configured to determine a concentration of a constituent (sample property) within the sample based on the measurement data provided to it by the detection unit 6, as will be described in more detail below, and to output the same Conc., for example in a human discernible format on a video display or in digital format for transmission, storage in a memory device or for use in other electronic systems.

The control unit 10 may be operably connected to the perturbations unit 14 to trigger the perturbations unit 14 to induce a perturbation in the liquid sample which causes time dependent variations in the degree of interaction between the output electromagnetic energy and the liquid sample which are associated with a component of the liquid sample which is related to the property of the sample being monitored. In the present example the control unit 10 may be also operably connected to the detection unit 6 to trigger the detection unit 6 to make detections for a plurality of times after the triggering of the perturbation by the perturbations unit 14. The thus generated time-series electromagnetic spectral data is the measurement data employed by the determinations unit 8 to determine a concentration of a component of interest within the liquid sample as the component related property of the sample.

The sampling unit 12 may include a perturbations unit 14 which may be operated to automatically induce a suitable perturbation in the liquid sample, for example directly in the sample held in the sample presentation unit 16. Here the perturbations unit 14 may operate to introduce a chemical, such as an enzyme for example, into the liquid sample to cause a reaction which manifests itself as a detectable change in the interaction of the liquid sample with the electromagnetic waves output from the output unit 4. The chemical/enzyme is selected to induce a change that is specific to the constituent related to the property of the sample to be determined, in accordance with the present invention the constituent concentration. Other perturbations may be substituted for a chemical one, dependent on the constituent. Such other perturbations may be of a thermal, electric or magnetic nature. The perturbations unit 14 may be manually operable and may for example be a pipette or syringe containing the appropriate chemical and may operate to introduce this chemical into the sample before it enters the sample presentation unit 16.

The sample presentation unit 16 of the sampling unit 12 may be configured according to the property of the electromagnetic wave being detected by the detecting unit 16. The sample presentation unit 16 may comprise opposing wall sections which are transparent to the electromagnetic wave output by the output unit 16 and may be formed as a removable cuvette into which a sample (with or without a perturbing chemical added) may be introduced manually. Alternatively, the sample presentation unit 16 may be formed as a flow-through cuvette having an inlet connected to a liquid flow system by which an external sample may be flowed into the cuvette for analysis, and an outlet through which analysed sample may be removed from the cuvette. An end of an externally accessible pipette of the liquid flow system may be immersed in a liquid to be analysed, for example as may be held in a beaker or a test tube, and the flow system may be operated to automatically introduce a liquid sample into the sample presentation unit 16. The perturbations unit 14 may be fluidly connected to the flow system to introduce a perturbing chemical/enzyme into liquid flowing towards the sample presentation unit 16.

According to an embodiment, in order to establish the empirical calibration for K-casein concentration reference samples were prepared using different dilutions of milk which is sourced from a consumer milk carton. According to the dilution the K-casein concentration was calculated simply by multiplying the total protein content (here obtained from milk package nutrition information but which may be obtained using standard analysis techniques such as a one employing Kjeldahl chemistry) with a factor of 0.8. It is generally accepted in the art, for example as evidenced in the text book: Mejeri-lære, authors E. Waagner Nielsen and Jens A. Ullum, ISBN 87-7510-536-5, pg. 62, that K-casein represents around 80% of total protein in milk.

The diluted milk reference samples employed in establishing the calibration are listed in Table 1 and are selected so that the K-casein concentration extend to cover the range of concentrations expected in samples of unknown K-casein concentrations to which the calibration will eventually be applied.

**Table 1**

| Sample Number | Milk (ml) | Water (ml) | Milk ratio | K-casein (g/100ml) |
|---|---|---|---|---|
| 1 | 25 | 5 | 0,83 | 2,33 |
| 2 | 24 | 6 | 0,80 | 2,24 |
| 3 | 23 | 7 | 0,77 | 2,15 |
| 4 | 22 | 8 | 0,73 | 2,05 |
| 5 | 21 | 9 | 0,70 | 1,96 |
| 6 | 20 | 10 | 0,67 | 1,87 |
| 7 | 19 | 11 | 0,63 | 1,77 |
| 8 | 18 | 12 | 0,60 | 1,68 |
| 9 | 17 | 13 | 0,57 | 1,59 |
| 10 | 16 | 14 | 0,53 | 1,49 |
| 11 | 15 | 15 | 0,50 | 1,40 |

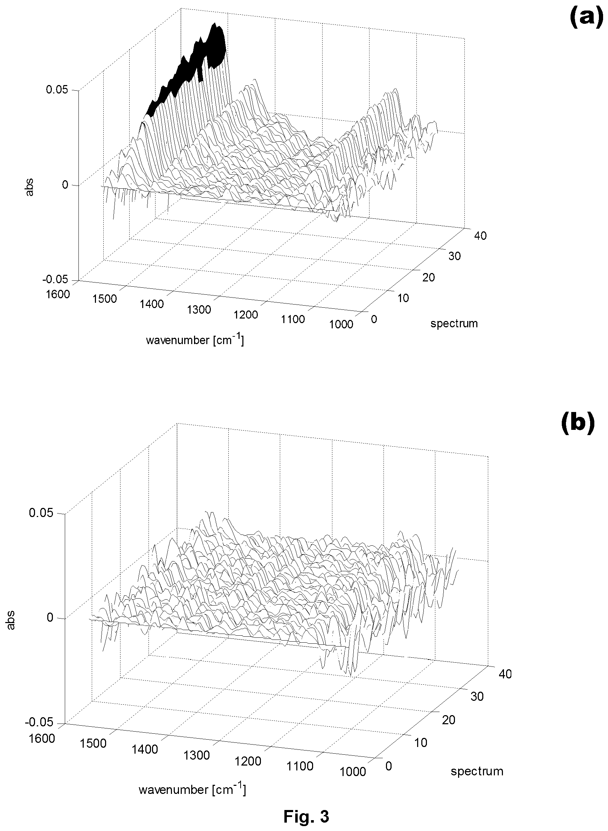

Temporal evolution profiles of the energy dependent absorption of the output electromagnetic wave which were recorded for reference samples identified by sample numbers 5 and 11 are presented in Figs 3 (a) and (b) respectively. As the acquisition time for each spectrum is known then the scan number illustrated in Figs. 3 represents a time scale for the collected spectral data. It can be clearly observed that the intensity of the spectral evolution for electromagnetic energy in the wavelength region between 6250nm (1600cm⁻¹) and 10000nm (1000cm⁻¹) increases with the K-casein concentration (i.e. increase from Fig 3(b) to Fig 3(a)) and increases with time after introduction of the perturbation/spectrum number (see Fig. 3 (a)). All perturbations have been induced by an introduction of 20µl of CHYMAXM1000 enzyme solution.

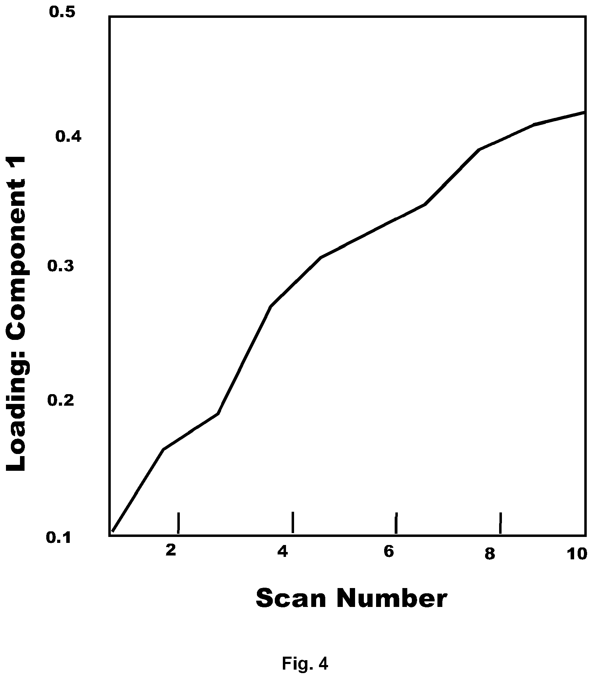

To establish a K-casein calibration multivariate chemometric, preferably multi-way, analysis methodology, such as for example PARAFAC, TUCKER3 or NPLS, are employed. Alternative multivariate chemometric methodologies are unfolded PLS, unfolded PCR, unfolded Ridge regression or similar, as well as variable selection techniques in connection with these methods (including Multivariate Linear Regression 'MLR'). For the true multi-way methodologies in principle only one calibration sample is sufficient to calibrate the system as opposed to the two way methods where several samples are needed to span the variations expected. In an embodiment PARAFAC is applied to the temporal evolution profiles in order to extract the underlying spectral patterns without reference data (which is unsupervised and therefore unbiased). PARAFAC is employed to decompose the tensor into sample mode, kinetic mode and spectral fingerprint mode. The retrieved PARAFAC kinetic mode is presented in Fig. 4 which shows a plot of Loading on PARAFAC Component 1 against scan number (equivalent to time after perturbation) and illustrates that the loadings on Component 1 increase with time (increase in scan number).

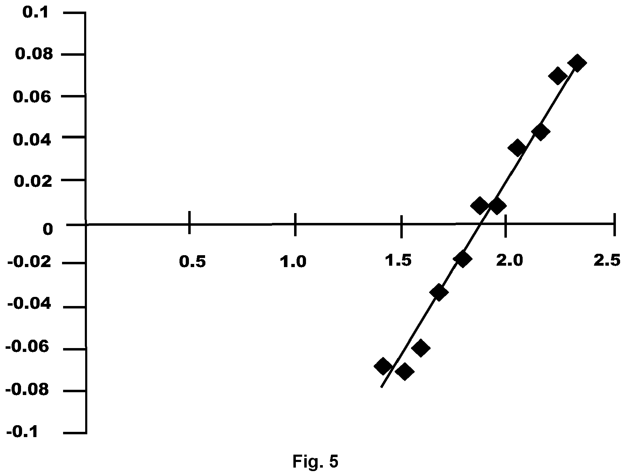

The final calibration was thereafter established by correlating the PARAFAC retrieved score for Component 1 with the K-casein concentration from Table 1. Calibration accuracy and precision were calculated for different models using different amounts of spectra (= different observation times). As shown in Figures 5, which is a plot of PARAFAC Component 1 scores against K-casein concentration in milk (g/100ml) for reference samples listed in Table 1, the K-casein concentration correlates well with the Component 1 scores retrieved by PARAFAC. Precision was determined by measuring nine replicates of one K-casein concentration.

For the present example the optimal observation time for good calibrations in terms of accuracy and precision are rather short. The PARAFAC model for an observation time of 1.9 min (7 spectra; each spectra having 16.6 seconds acquisition time) showed best performance.

To determine a K-casein concentration in a test sample of unknown concentration a time-series of electromagnetic spectra in the wavelength region between 6250nm (1600cm⁻¹) and 10000nm (1000cm⁻¹) are recorded for this test sample essentially in the same manner as the time-series were obtained for the reference samples. Generally then, the enzyme is added to the test sample and electromagnetic spectra including the wavelength region between 6250nm (1600cm⁻¹) and 10000nm (1000cm⁻¹) are recorded by a sample property monitor equivalent to that monitor 2 used to generate the model and illustrated in Fig. 2. By the term 'equivalent' it is to be understood to mean a sample property monitor which would generate spectral data from a sample that would be detectably indistinguishable from spectral data generated using the monitor employed in calibration generation. These spectra are obtained over the same observation time and acquisition time as employed in the derivation of the calibration model stored in the system. The recorded time-series data may be subjected to PARAFAC decomposition in order to obtain a score for the Component 1, the stored calibration model is then applied to the obtained score in the concentration determining unit 8 and a concentration (amount) of K-casein in the test sample is obtained,

When more than one component is required a multivariate chemometric regression method such as MLR may be employed to correlate these components with the sample property of interest.

In a further embodiment of the method according to the present example proteins are detected in a multi-component food sample, such as milk or an emulsified solid food product. Here, the technique of protein denaturation is employed as the perturbation. Protein denaturation is any modification in conformation not accompanied by the rupture of peptide bonds involved in primary structure and according to the present invention it is the temporal evolution in conformation modifications that is monitored. Heat, acids, alkalis, concentrated saline solutions, solvents and electromagnetic radiations are all perturbations which are known to cause denaturation. It is known to monitor protein denaturation by measuring sedimentation; viscosity; protein migration through electrophoresis; optical rotary dispersion, dichroism, wavelength dependent intensity variations or other changes in a property of an electromagnetic wave. Thus according to the present invention a suitable perturbation is induced in a test sample and a time-series of measurement data is recorded. A suitably derived empirical calibration correlating the constituent (protein) related sample property with changes in time-series of measurement data is applied in a data processor to the recorded time-series of measurement data in order to determine the constituent related property of the sample,: being the concentration in accordance with the present invention, for the test sample. Such an empirical calibration may be generated in a manner analogous to that described above in respect of the K-casein calibration. Thus, time-series measurement data is obtained for a plurality of reference samples having known amounts of the protein of interest using the same methodology as employed in obtaining the time-series measurement data of the test sample. Multivariate chemometric modelling, such as multi-way modelling described above, is applied to the time-series measurement data for each reference sample in order to generate the calibration.

It will be appreciated that calibrations linking temporal evolution measurement data with other sample properties of interest can be generated by using reference samples having known values of that sample property and subjecting temporal evolution profiles of these reference samples to a multivariate chemometric modelling.

2. An example of a sample property monitor not in accordance with the present invention which may comprise a sampling unit 812 is illustrated in Fig. 8 which substitutes for the sampling unit 12 of Fig. 2 in order to adapt the component analyser 2 of Fig. 2 to operate to determine a concentration (Cone.) of a constituent of interest in a multi-constituent liquid sample using an electrophoretic perturbation. The sampling unit 812 may comprise a perturbations unit 814 and a sample presentation unit 816.

The perturbations unit 814 may be a direct current (D.C.) generator operably connected to the sample presentation unit to generate an electric field within a multi-constituent liquid sample held in the sample presentation unit 816.

The sample presentation unit 816 may be by way of example only, configured as a flow-through cuvette provided with liquid inlet 818 and liquid outlet 820 in fluid connection with a liquid flow system (not shown) of the sample property monitor. The flow-through cuvette 816 has opposing window portions 822 and 824(dashed) which are formed of a material transparent to electromagnetic energy output from the output unit 4 and dimensioned to provide an observation region (shaded region) within the cuvette 816 which is less than the liquid holding region of the cuvette.

In use the perturbations unit 814 may generate a D.C. electric field within liquid sample in the liquid holding region (which liquid holding region is the entire liquid holding volume of the sample presentation unit 816) which causes charged constituents of the liquid sample to move through the observation region 826 towards either the negative (-) or positive (+) poles of the cuvette 816, in a direction dependent on their charge. A plurality of electromagnetic spectra are recorded at different times after application of the electric filed by the perturbations unit 814 and this time-series of electromagnetic spectral data is employed in the determinations unit 8 to determine a concentration (amount) or the presence/absence of a constituent of the multi-constituent liquid sample and to output the same (Conc.). A calibration is obtained empirically from the application of multivariate chemometric modelling to time-series reference sample data. This calibration, which thus correlates a sample property to be determined with features in the time-series of electromagnetic spectral data, is made available for use, for example stored within an accessible electronic memory or other storage device, within the determinations unit 8 for this purpose. This calibration is derived in basically the same manner as that for K-casein described above. In the present embodiment however, and as will be appreciated by the skilled artisan, temporal evolution profiles are recorded for reference samples of known concentration of the constituent not consequent on the addition of an enzyme but, rather, consequent on the application of the D.C. electric field. Moreover the output unit 4 may be adapted to output the electromagnetic wave in an appropriate region of the electromagnetic spectrum, which is sensitive to the electrical perturbation induced changes to the constituent. As mentioned above a sample property monitor may be applied to the determination of protein related information as the sample property of interest through monitoring time related changes which are manifestations of the denaturation of the protein of interest.

## Claims

1. A method of determining a concentration of a constituent of a multi-constituent sample comprising: subjecting the sample to a perturbation affecting only the constituent and selected to induce a time dependent change in a wavelength dependent intensity of an electromagnetic wave after its interaction with the sample, the electromagnetic wave having wavelength components, the intensities of which change dependent on the perturbation induced time dependent change; recording a time-series of the wavelength dependent intensity of the electromagnetic wave following subjecting the sample to the perturbation; and determining the concentration of the constituent from the application of a calibration to the recorded time-series, said calibration correlating the concentration with time-series of the wavelength dependent intensity of the electromagnetic wave and being empirically derived from multivariate chemometric modelling of time-series wavelength dependent intensities of the electromagnetic wave recorded for each of a plurality of reference samples following subjecting each reference sample to the perturbation, each reference sample having a different known value of the concentration of the constituent.

2. A method as claimed in claim 1 wherein the multivariate chemometric modelling comprises the application of a multi-way modelling methodology to the time-series measurement data.

3. A method as claimed in Claim 2 wherein the multi-way modelling methodology is a methodology selected from PARAFAC, TUCKER3 and NPLS.

4. A method as claimed in any of the claims 1 to 3 wherein subjecting the sample to a perturbation consists of subjecting the sample to a chemical perturbation.

5. A method as claimed in Claim 4 wherein the chemical perturbation comprises introducing an enzyme to the sample.

6. A method as claimed in Claim 4 or Claim 5 wherein the constituent is a protein in a food sample.

7. A method as claimed in Claim 6 wherein the sample is milk and the chemical is an enzyme selected to facilitate K-casein agglomeration and wherein the step of determining the concentration of the constituent comprises determining a concentration of K-casein.

8. A method as claimed in Claim 7 wherein the enzyme is a chymosin preparation.

9. A method as claimed in claims 1 to 3 wherein subjecting the sample to a perturbation consists of subjecting the sample to a physical perturbation.

10. A method as claimed in Claim 9 wherein the physical perturbation is one or other of an electric perturbation and a magnetic perturbation selected to create a movement of dispersed particles within the multi-constituent sample.

## Patentansprüche

1. Verfahren zum Bestimmen einer Konzentration eines Bestandteils einer Probe mit mehreren Bestandteilen, umfassend: Aussetzen der Probe einer Störung, die sich nur auf den Bestandteil auswirkt, und ausgewählt ist, um eine zeitabhängige Änderung einer wellenlängenabhängigen Intensität einer elektromagnetischen Welle nach ihrer Wechselwirkung mit der Probe zu induzieren, wobei die elektromagnetische Welle Wellenlängenkomponenten aufweist, deren Intensitäten sich abhängig von der störungsinduzierten zeitabhängigen Änderung ändern;
Aufzeichnen einer Zeitreihe der wellenlängenabhängigen Intensität der elektromagnetischen Welle, nachdem die Probe der Störung ausgesetzt wurde; und
Bestimmen der Konzentration des Bestandteils aus der Anwendung einer Kalibrierung auf die aufgezeichneten Zeitreihen, wobei die Kalibrierung die Konzentration mit Zeitreihen der wellenlängenabhängigen Intensität der elektromagnetischen Welle korreliert und aus einer multivariaten chemometrischen Modellierung von wellenlängenabhängigen Intensitäten der Zeitreihen der elektromagnetischen Welle empirisch abgeleitet wird, die für jede von mehreren Referenzproben aufgezeichnet werden, nachdem jede Referenzprobe der Störung ausgesetzt wurde, wobei jede Referenzprobe einen unterschiedlichen bekannten Wert der Konzentration des Bestandteils aufweist.

2. Verfahren nach Anspruch 1, wobei die multivariate chemometrische Modellierung die Anwendung einer Mehrwegmodellierungsmethodik auf die Zeitreihenmessdaten umfasst.

3. Verfahren nach Anspruch 2, wobei die Mehrwegmodellierungsmethodik eine Methodik ist, die aus PARAFAC, TUCKER3 und NPLS ausgewählt ist.

4. Verfahren nach einem der Ansprüche 1 bis 3, wobei das Aussetzen der Probe einer Störung darin besteht, die Probe einer chemischen Störung auszusetzen.

5. Verfahren nach Anspruch 4, wobei die chemische Störung ein Einbringen eines Enzyms in die Probe umfasst.

6. Verfahren nach Anspruch 4 oder 5, wobei der Bestandteil ein Protein in einer Lebensmittelprobe ist.

7. Verfahren nach Anspruch 6, wobei die Probe Milch ist und die Chemikalie ein Enzym ist, das ausgewählt ist, um eine Agglomeration von K-Casein zu ermöglichen, und wobei der Schritt des Bestimmens der Konzentration des Bestandteils das Bestimmen einer Konzentration von K-Casein umfasst.

8. Verfahren nach Anspruch 7, wobei das Enzym ein Chymosinpräparat ist.

9. Verfahren nach den Ansprüchen 1 bis 3, wobei das Aussetzen der Probe einer Störung darin besteht, die Probe einer physikalischen Störung auszusetzen.

10. Verfahren nach Anspruch 9, wobei die physikalische Störung die eine oder andere einer elektrischen Störung und einer magnetischen Störung ist, die ausgewählt sind, um eine Bewegung dispergierter Teilchen innerhalb der Probe mit mehreren Bestandteilen zu erzeugen.

## Revendications

1. Procédé de détermination d'une concentration d'un constituant d'un échantillon à plusieurs constituants comprenant : la soumission de l'échantillon à une perturbation affectant uniquement le constituant et sélectionnée pour induire un changement dépendant du temps d'une intensité dépendant de la longueur d'onde d'une onde électromagnétique après son interaction avec l'échantillon, l'onde électromagnétique ayant des composantes de longueur d'onde, dont les intensités changent en fonction du changement dépendant du temps induit par la perturbation ; l'enregistrement d'une série temporelle de l'intensité dépendant de la longueur d'onde de l'onde électromagnétique après avoir soumis l'échantillon à la perturbation ; et la détermination de la concentration du constituant à partir de l'application d'un étalonnage à la série temporelle enregistrée, ledit étalonnage corrélant la concentration avec la série temporelle de l'intensité dépendant de la longueur d'onde de l'onde électromagnétique et étant empiriquement dérivé d'une modélisation chimiométrique multivariée des intensités dépendant de la longueur d'onde en série temporelle de l'onde électromagnétique enregistrées pour chacun d'une pluralité d'échantillons de référence après avoir soumis chaque échantillon de référence à la perturbation, chaque échantillon de référence ayant une valeur connue différente de la concentration du constituant.

2. Procédé selon la revendication 1, la modélisation chimiométrique multivariée comprenant l'application d'une méthodologie de modélisation multivoies aux données de mesure en série temporelle.

3. Procédé selon la revendication 2, la méthodologie de modélisation multivoies étant une méthodologie sélectionnée parmi PARAFAC, TUCKER3 et NPLS.

4. Procédé selon l'une quelconque des revendications 1 à 3, la soumission de l'échantillon à une perturbation consistant à soumettre l'échantillon à une perturbation chimique.

5. Procédé selon la revendication 4, la perturbation chimique comprenant l'introduction d'une enzyme dans l'échantillon.

6. Procédé selon la revendication 4 ou la revendication 5, le constituant étant une protéine dans un échantillon alimentaire.

7. Procédé selon la revendication 6, l'échantillon étant du lait et le produit chimique étant une enzyme sélectionnée pour faciliter l'agglomération de la caséine K et l'étape de détermination de la concentration du constituant comprenant la détermination d'une concentration de caséine K.

8. Procédé selon la revendication 7, l'enzyme étant une préparation de chymosine.

9. Procédé selon les revendications 1 à 3, la soumission de l'échantillon à une perturbation consistant à soumettre l'échantillon à une perturbation physique.

10. Procédé selon la revendication 9, la perturbation physique étant l'une ou l'autre d'une perturbation électrique et d'une perturbation magnétique sélectionnées pour créer un mouvement de particules dispersées à l'intérieur de l'échantillon à plusieurs constituants.
